Europäisches Patentamt

European Patent Office

Office européen des brevets

(11) Publication number: **0 061 331**
**B1**

# (12) EUROPEAN PATENT SPECIFICATION

(45) Date of publication of patent specification: **22.01.86**

(51) Int. Cl.⁴: **A 61 B 10/00, A 61 B 8/12**

(21) Application number: **82301452.7**

(22) Date of filing: **22.03.82**

(54) **Combined endoscope and ultrasonic diagnostic device.**

(30) Priority: **22.03.81 JP 40819/81**
**22.03.81 JP 40820/81**
**22.03.81 JP 40822/81**
**22.03.81 JP 40823/81**

(43) Date of publication of application:
**29.09.82 Bulletin 82/39**

(45) Publication of the grant of the patent:
**22.01.86 Bulletin 86/04**

(84) Designated Contracting States:
**AT BE CH DE FR GB IT LI NL SE**

(56) References cited:
**EP-A-0 028 825**
**EP-A-0 037 047**
**DE-A-3 009 482**
**FR-A-2 396 295**
**FR-A-2 467 583**
**GB-A-2 048 079**
**US-A-3 269 387**

(73) Proprietor: **OLYMPUS OPTICAL CO., LTD.**
**43-2, 2-chome, Hatagaya Shibuya-ku**
**Tokyo 151 (JP)**

(72) Inventor: **Matsuo, Kazumasa**
**No. 1-7-304, Ochiai 3-chome**
**Tama-shi Tokyo (JP)**
Inventor: **Nakada, Akio**
**No. 13-33, Hiyoshi-cho**
**Hachioji-shi Tokyo (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

Courier Press, Leamington Spa, England.

**Description**

This invention relates to combined endoscope and ultrasonic diagnostic devices for the examination of internal body cavities, wherein the endoscope is incorporated in the housing of an ultrasonic probe in such a manner that the probe tip can be held to contact and diagnose an internal organ whilst being viewed via the endoscope.

As a means of obtaining information on an object ultrasonic wave probes have recently come to be extensively used in the medical field. For example, when ultrasonic wave pulses are projected into a body from its surface, the projected waves will be propagated on to be reflected by any discontinuous boundary surface forming an acoustic impedance represented by the product of the density of the medium and the viscosity of the sound, and therefore the reflected pulses can be received and information for use in diagnosis obtained.

As compared with an X-ray device, such ultrasonic wave diagnostic devices have the advantages that information on a soft biotic structure can be easily obtained without requiring aids such as x-ray opaque substances or contrast media, that a biotic structure is not destroyed by high energy rays, and that the diagnostic device is easier to handle and is less dangerous. Recent developments in ultrasonic techniques have improved the quality and quantity of the obtained information to such a degree that the ultrasonic probe is becoming popular for use as a clinical diagnostic device.

In comparison with the above mentioned diagnosis by the use of ultrasonic wave pulses transmitted and received from the surface of a body, the method of diagnosis in a body cavity by the use of ultrasonic wave pulses transmitted and received from a position near an internal biotic organ within that body cavity has advantages, in that it is possible to obtain readily analysable high frequency information of high precision, and the diagnosis is not influenced by any hypodermic fat layer or the like that may be interposed, and therefore such probes will be used more in the future. Such body cavity interior ultrasonic wave diagnostic devices, constructed to be used when inserted into a body cavity, generally incorporate an endoscope as a convenient optical observation means.

Known combinations of such diagnostic devices incorporating both an ultrasonic probe and an endoscope include the following disclosures:

"Probe for Inspecting a Body Cavity Interior" mentioned in the Gazette of Japanese Patent Laid Open No. 94230/1980;

"Body Cavity Interior Inspecting Probe" mentioned in the Gazette of Japanese Patent Laid Open No. 94231/1980;

"Ultrasonic Wave Probe" mentioned in the Gazette of Japanese Patent Laid Open No. 96130/1980;

"Endoscope" mentioned in the Gazette of Japanese Patent Laid Open No. 96132/1980; and

"Endoscope" as described in the German Offenlegungschrift No. 30 09 482 published on the 18th September 1980.

However, the above-mentioned prior art discloses nothing with regard to the optimum arrangements with regard to a driving cable for the ultrasonic wave oscillator or scanning mirror, a signal cable for the ultrasonic wave oscillator, or a light guide cable connected with a light source means to transmit illuminating light to the endoscope.

Our European Patent Application EP—A— 0 028 825 describes and claims an ultrasonic scanner with optical means, in which at least two of an insertion body, beam-transmitting and receiving section and beam-scanning section are detachably attached by connecting means.

One object of the present invention is to provide a combined endoscope and ultrasonic diagnostic device for the examination of the interior of a body cavity, provided with a grip for the inserted part which can be made small and light, to facilitate insertion and operation, and improve diagnosis, operation only requiring the operator to grip with one hand, and therefore enabling the operator to do other work such as adjusting the posture of the patient and pressing the body wall for example. This may be advantageously achieved if the above-mentioned cable to the grip and the light guide cable to the endoscope are connected from the same lateral direction, such that in operation, e.g. the process of insertion and the step of contacting, they can be prevented from causing any interference due to any touching of the body wall, etc.

The invention provides a combined endoscope and ultrasonic diagnostic device wherein the endoscope, provided with illuminating light from a light source via a flexible light guide coupling connected to extend laterally from the endoscope, is contained, within an elongate insertion sheath carrying an ultrasonic wave probe for inserting directly into a body cavity and transmitting and receiving ultrasonic waves to facilitate diagnosis within the body cavity, the endoscope entering up to a bendable curving part, and the probe extending beyond the bendable curving part and being provided with an ultrasonic oscillator and a scanning mirror, the oscillator being connected via a signal lead to drive means in a hand grip adjacent the proximal end of the device, and the scanning mirror being mounted on a rotary drive shaft extending to the hand grip, on which is provided a control member with a thumb-operable curvature control lever, characterised in that separate driving means for the oscillator and scanning mirror are coupled to the control member via a flexible drive cable coupling, whereby diagnosis can be facilitated by optical observation via the endoscope whilst using the ultrasonic probe, the flexible couplings enabling the hand grip itself to be relatively small and light, thereby improving the degree of positional control and reducing operator fatigue, and the flexible drive cable coupling being connected to the control member from the same lateral direction as the flexible light guide coupling is connected to the endoscope from the light source, to facilitate insertion and operation of the device.

The invention will now be described with reference to the drawings, in which:

Figure 1 is a side view of one known combined endoscope and ultrasonic diagnostic device;

Figure 2 is an explanatory view of the diagnostic device of Figure 1 in use;

Figure 3 is a simplified perspective view of one exemplary embodiment of the present invention;

Figure 4 is a side view of the endoscope portion pulled out of a probe housing that forms an insertion sheath;

Figure 5 is a view of the sheath probe tip seen in the direction indicated by an arrow A in Figure 4;

Figure 6 is a cross-sectional view of the probe tip and curved part;

Figure 7 is a sectional view showing details of an incised window in the insertion sheath and structure of the curved part and a connecting member;

Figure 8 is a cross-section on the line B—B in Figure 7;

Figure 9 is a cross-section of the insertion sheath;

Figure 10 is a fragmentary section showing details of a hand grip and associated housing parts;

Figure 11 is an explanatory view showing the device in use;

Figure 12 is an explanatory view showing the field of view of the endoscope in Figure 11;

Figure 13 is a detailed section showing the interior of the operating part;

Figure 14 is a section showing the arrangement of a driving cable on the hand grip side;

Figure 15 is a section showing the arrangement of a driving cable on the driving means unit side; and

Figure 16 is a section showing a connector for connecting the driving means unit.

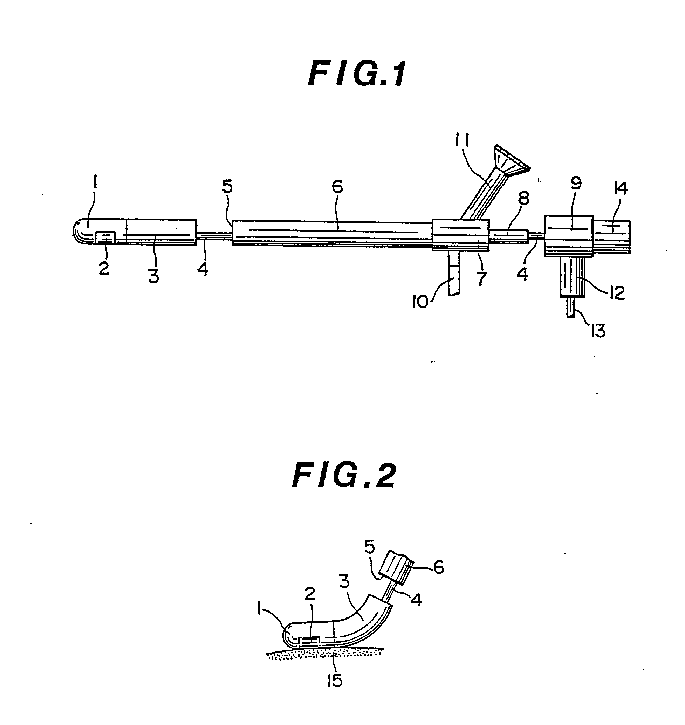

Prior to explaining an embodiment of the present invention, we will describe a known conventional example of a diagnostic device combining an endoscope for observing a body cavity interior and an ultrasonic probe for obtaining a signal image, shown in Figures 1 and 2. In these drawings, an ultrasonic probe tip 1 has a window 2 for transmitting and receiving ultrasonic waves formed in a side of the tip, and a fine diameter shaft 4 is connected to the rear of the tip through a curving part 3. Leads for transmitting and receiving ultrasonic signals and cables (not illustrated) for driving a mirror within the above mentioned probe tip 1 are contained in this shaft. This shaft 4 is passed through a channel in an insertion sheath 6 which also houses an endoscope 5 and is connected at its rear end to a driving unit 9 through an endoscope hand grip 7 and channel inserting port 8. A light guide cable 10 and an eyepiece 11 inclined to the axial direction of the insertion sheath 6 are connected to the above-mentioned endoscope hand grip 7. A handle member 12 is connected below the driving unit 9. A signal cable 13 is inserted through the handle member 12. A driving motor 14 is connected to the rear of the driving unit 9.

In the above mentioned diagnostic device, in order to obtain an ultrasonic wave image, the insertion sheath 6 and probe tip 1 are inserted into a body cavity, the probe tip 1 is pressed down in contact with a predetermined internal organ 15 that is in field of vision of the endoscope 5 within the insertion sheath 6 and the curving part 3 is bent as shown in Figure 2 so as to bring the window 2 of the probe tip 1 into contact with the internal organ 15.

The above mentioned formation has the disadvantages that during operation, the operator must hold the handle member 12 of the driving unit 9 by one hand whilst holding the endoscope hand part 7 with the other hand, and therefore the operator cannot do other tasks, such as adjusting the posture of the patient and/or pressing the body wall, for example.

Furthermore, as the driving unit 9 and driving motor 14 are integrally arranged on the handle member 12 of the insertion sheath 6 the handle member 12 is so heavy that it is hard to insert the sheath 6 into a body cavity and carry out a diagnostic operation. In addition, the large volume on the hand side interferes with the operation of the device and observation via the endoscope.

As the signal cable 13 is connected to the underside of the handle member 12, then particularly if the insertion sheath 6 is inserted deeply to diagnose a remote internal organ, the inclination of the sheath 6 is large, and the signal cable 13 may contact the body wall and interfere with the operation, which is most disadvantageous.

Yet another disadvantage of the conventional device, is that the light guide 10 of the endoscope 5 and the signal cable 13 of the driving unit 9 are connected respectively to the endoscope 5 and the handle member 12 from different directions, and this can interfere with operations such as inserting the sheath 6 into the body cavity, causing the curving part 3 to curve, or effecting a close contact between the probe tip 1 and an internal organ.

It will be readily appreciated that the most desirable requirement is to simplify the operator's task in conducting such diagnostic examinations, since this will not only reduce operator fatigue, but will assist in the operator being able to concentrate to the fullest extent on the information that is being obtained optically or via the ultrasonic scanning system, and furthermore enables swift diagnosis with consequential benefit to the patient who is undergoing examination.

Furthermore, as a result of the greater efficiency it becomes possible to deal with a greater number of patients in a given clinical examination period.

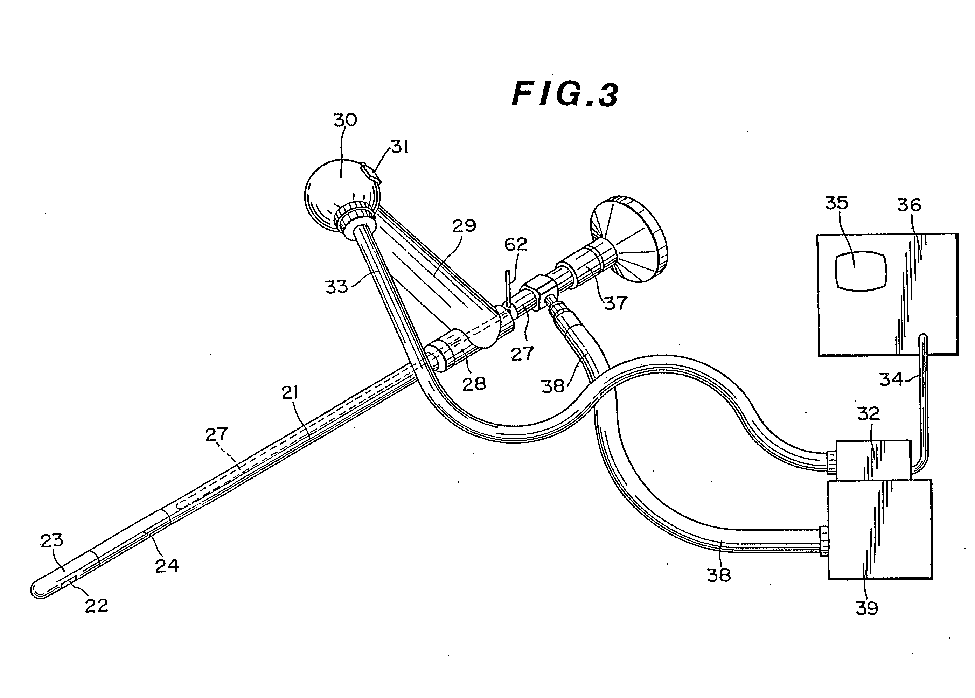

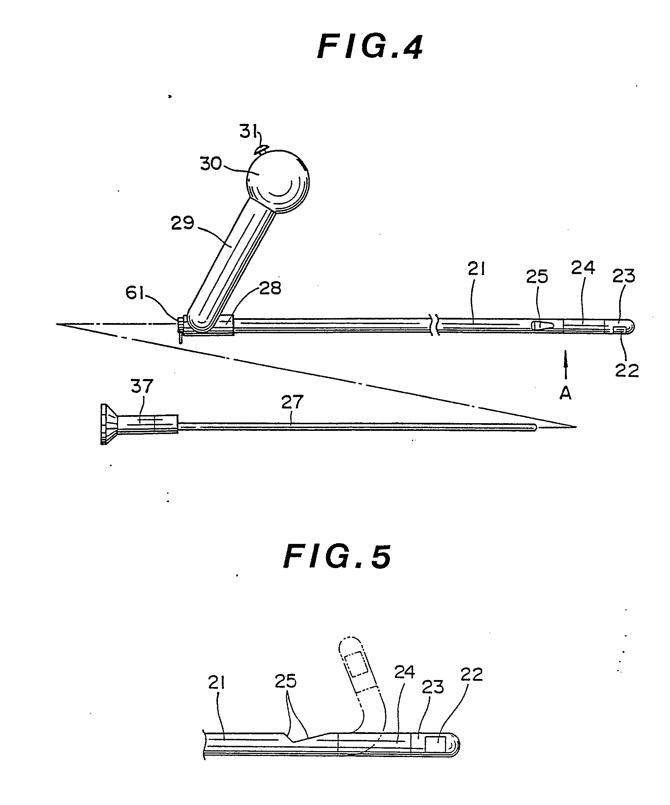

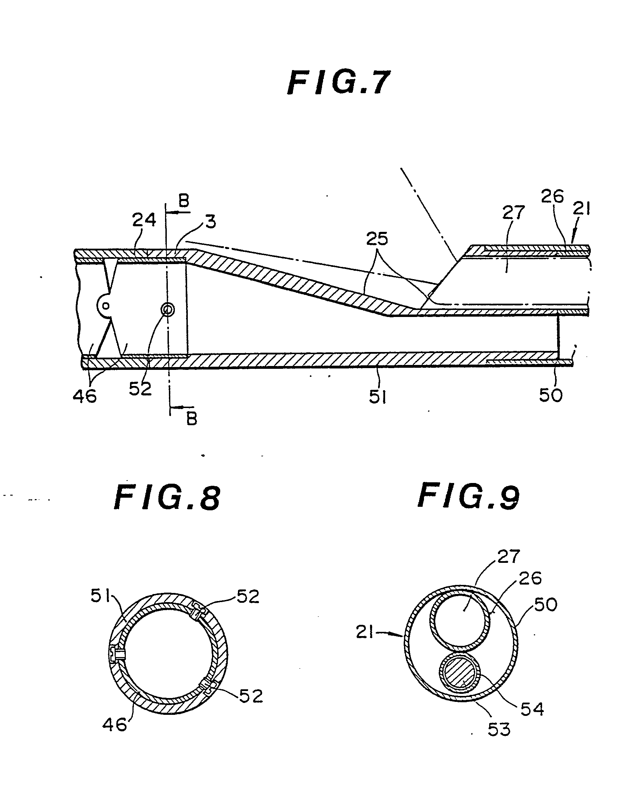

An exemplary embodiment of a device constructed in accordance with the present invention will now be described with reference to Figures 3 to 12. In these drawings, an insertion sheath 21 carries a probe tip 23 having a window 22 for transmitting and receiving ultrasonic waves on a side face, and a curving part 24 connects the front of the insertion sheath 21 to the probe tip 23, the sheath 21, probe tip 23 and curving part 24 all being substantially equal in diameter. An inset window 25 (Figure 4, Figure 7) is provided in the outer periphery of the insertion sheath 21 to the rear of the curving part 24 to give a field of vision in a partly lateral direction, and an endoscope guide tube 26 is arranged to extend from this inset window 25 back to the rear end of the insertion sheath 21. A perspective endoscope 27 of a fine diameter is telescopically inserted into the guide tube 26 in this embodiment. The illustrated device is an abdominal cavity internal organ diagnostic device, and the inset window 25 formed in the insertion sheath 21 directed toward the right, as seen from the operator in the normal operating state, so that the tip of the perspective endoscope 27 may be positioned in the field of view of this inset window 25 of the endoscope. The above-mentioned perspective endoscope 27 has a field of view with an included angle of at least 45°. The inset window 25 is formed in the sheath 21 to give the edge of the field of view an angle of inclination of 5° to 25° with respect to the longitudinal axis, so that the field of view of the endoscope 27 is not intercepted by the outer periphery of the insertion sheath 21.

A hand grip mount 28 is connected to the rear end of the insertion sheath 21. A grip 29 inclined forwardly at an angle of about 60° with respect to the longitudinal axis is integrally connected to the outer periphery of the upper part of this hand grip mount in the normal operating state, so that it can be gripped with one hand by an operator, and so that the grip 29 will not contact the body wall and will not obstruct the manipulation of the device, even if the insertion sheath 21 needs to be much inclined during insertion into a body cavity to diagnose a remote internal organ. The head of this grip 29 has a substantially spherical curve-control member 30 on which a curvature operating lever 31 is provided to project so as to be operable by the thumb when a hand is grasping the grip 29.

A driving means 32 containing a motor for producing a scanning motion of ultrasonic waves is formed separately from the grip 29, and is connected thereto via a flexible drive cable 33. Further, the driving means 32 is connected by a signal cable 34 to an indicating means 36 provided with an oscilloscope 35. A flexible light guide 38 is connected in front of an eyepiece 37 of the perspective endoscope 27 to feed in light from a light source 39. This light guide 38 and the drive cable 33 are connected in the same lateral direction with respect to the eyepiece 37 of the endoscope and the curve-control member 30 at the head of the grip 29, to improve operatability and avoid any interference during insertion of the sheath 21 into the body cavity, or the contacting operation placing the probe tip 23 against a predetermined internal organ for the diagnosis using ultrasonic waves.

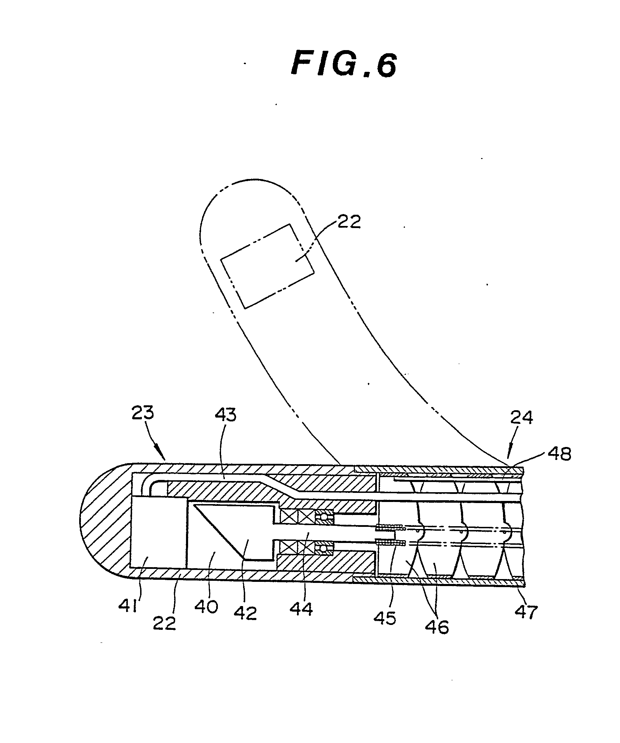

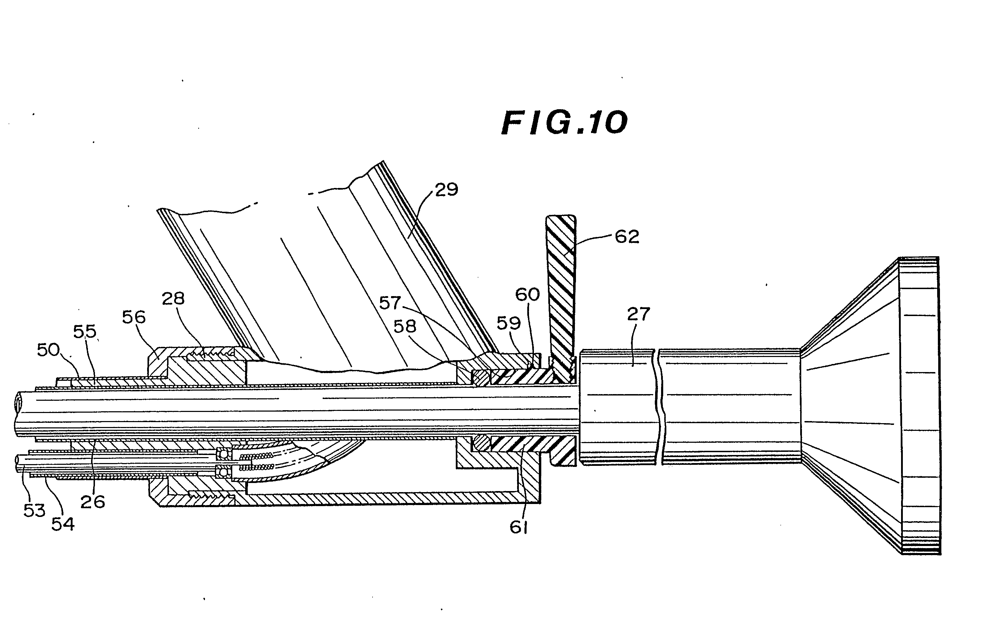

The probe tip 23 and curving part 24 are formed as shown in Figure 6, a medium chamber 40 is formed in the ultrasonic wave probe tip 23. An ultrasonic oscillator 41 and a mirror 42 are arranged opposed each other within a transmission medium chamber 40. A signal lead 43 extending through the insertion sheath 21 from the driving means 32 is connected to this oscillator 41. A shaft 44 is formed on the mirror 42. A flexible drive 45 is connected between this shaft 44 and an intermediate drive shaft 53, which is shown in Figure 10. The curving part 24 is formed by pivoting together a plurality of articulated members 46 which are each provided with a rubber coating 47. An operating wire 48 inserted through the curvature control member 30 is fixed to the foremost articulated member 46.

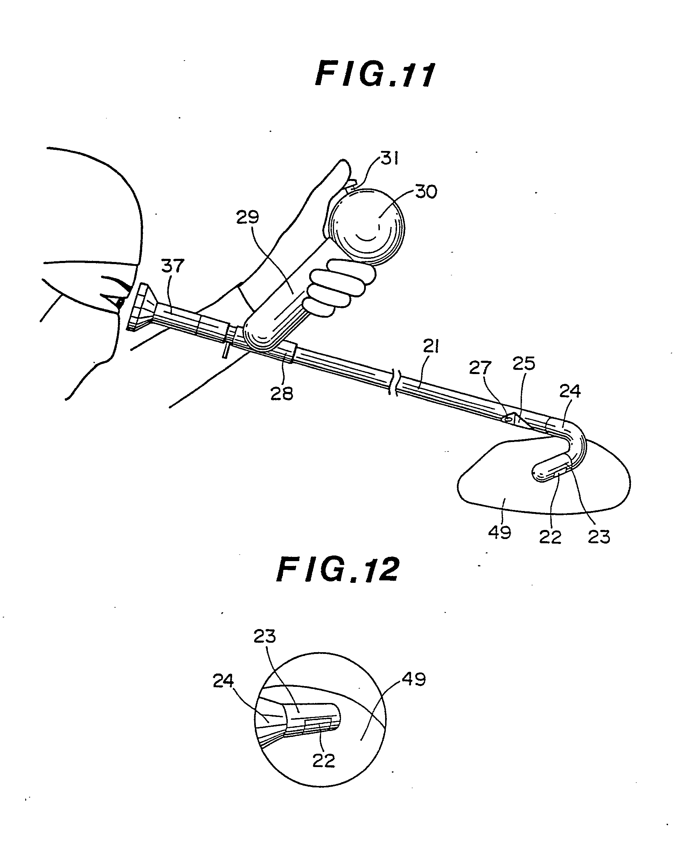

The bending direction of this curving part 24 is set to lie in the same lateral direction as the field of view of the endoscope 27, that is, to the right as seen by the operator in the normal operational state, so that the window 22 which serves for transmitting and receiving ultrasonic waves may be within the field of view of the endoscope 27, as shown in Figure 12. Thus, in the illustrated device for diagnosing internal organs within the abdominal cavity, the direction of the field of view of the endoscope 27 inserted in the insertion sheath 21 and the bending direction of the curving part 24 are set to the right as seen from the operator because, in the case of using the device to perform a diagnosis on an internal organ 49 within an abdominal cavity, that part of the skin containing many blood vessels and that position at which the internal organ is located are naturally known, and therefore the inserting position and inserting direction are medically predetermined, and with the field of view to the right in the normal operating state and with the curving part bent in the direction of the field of view, the device will be adapted to operate to permit observation of the internal organ and the close contact of the probe tip 23 with the internal organ. If the device is to be used to diagnose internal organs other than those within the abdominal cavity, then the direction of the field of view of the endoscope and of the curved direction need not be to the right, considered from the operator, provided always that the window 22 in the probe tip 23 is arranged at the same side as the endoscope window 25, and the direction of bending of the curving part is in the same direction.

As shown in Figure 7, the inset window 25 formed in the insertion sheath 21 is in a connecting member 51 connecting the tip of an outer tube 50 forming part of the insertion sheath 21 to the curving part 24. This connecting member 51 is fitted and fixed to the inner face of the outer tube 50, and at its end adjacent the curving part 24 is secured to the last articulated member 46 by screws 52 screwed in from the outer periphery as shown in Figure 8. This formation ensures that there is the widest possible space inside the connecting part of the connecting member 51 available to accommodate the intermediate drive shaft 53, signal cable 43 and operating wire 48, together with each articulated member 46. To accommodate the inset window 25 formed in the sheath 21, the mirror driving shaft 53 inserted through the outer tube 50 of the sheath is arranged on the side opposite the direction of the field of view of the endoscope 27, as shown in Figure 9.

Further, the connection of the sheath 21 to hand grip mount 28 is as shown in Figure 10. A pipe receiver 55 holding and fixing the endoscope guide tube 26, drive shaft tube 54 and the sheath outer tube 50 is fitted within the hand grip mount 28, a male screw thread is formed on the outer periphery of this hand grip body 28 and a cap 56 having a female screw thread engages on the male screw thread to secure the pipe receiver 55 to the hand grip mount 28. A fixing hole 57 communicating with the endoscope guide tube 26 is formed at the rear of the hand grip mount 28. An O-ring 58 engaging with the outer periphery of the endoscope 27 is inserted in this fixing hole 57 and secured in place by an O-ring fastening body 61 having a projecting pin 60 engaged in a spiral groove 59 provided on the inner periphery within the hole 57, so that the body 61 can be screwed into the fixing hole 57 to press and deform the O-ring 58. The endoscope 27 is held in place by this deformed O-ring to prevent axial or rotating movement and form an airtight seal. The ring fastening body 61 is formed to receive the endoscope 27 and is provided with a lever 62 that projects outwardly at the rear.

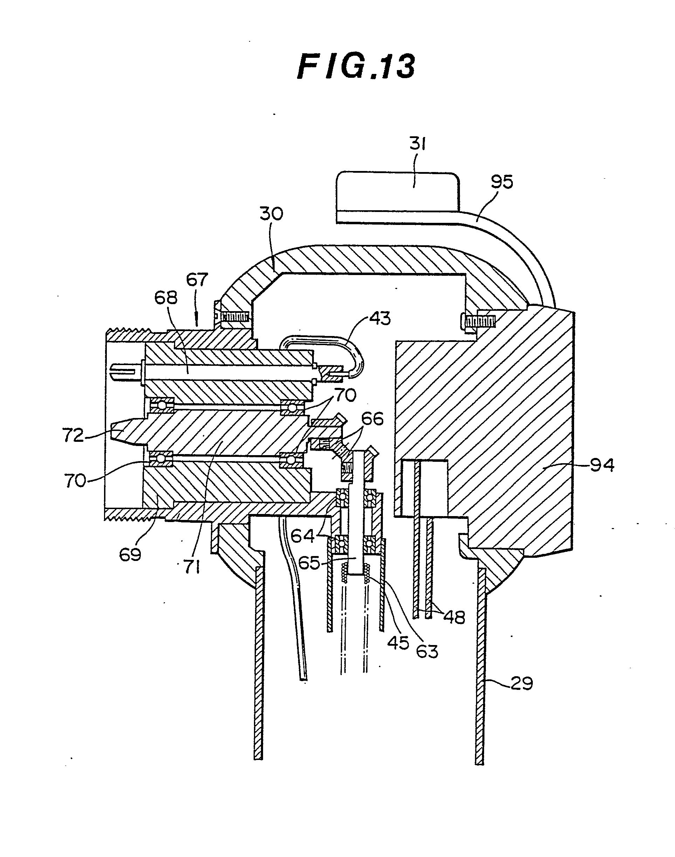

As shown in Figure 13, the flexible drive shaft 45 arranged within the grip 29 is contained within a flexible tube 63 and is inserted in the axial direction through the grip 29. This flexible drive shaft 45 is connected at the upper end with a shaft 65 carried in bearings 64 at the head of the grip 29 to drive a bevel gear 66 pivoted to the upper end of this shaft 65. A lateral connector 67 is formed in the control member 30, is borne by bearings 70 containing an annular insulating member 69 through which male connecting pins 68 are inserted (of which one is shown), to connect the signal lead 43 to the probe tip. Within the insulating member 69 there is supported a drive shaft link 71 that is driven via the bevel gear 66, the drive shaft link 71 is normal to the shaft 65. A hexagonal projection 72 is formed as an output connector for the drive shaft link 71. A male screw thread is formed on the outer periphery of the connector 67.

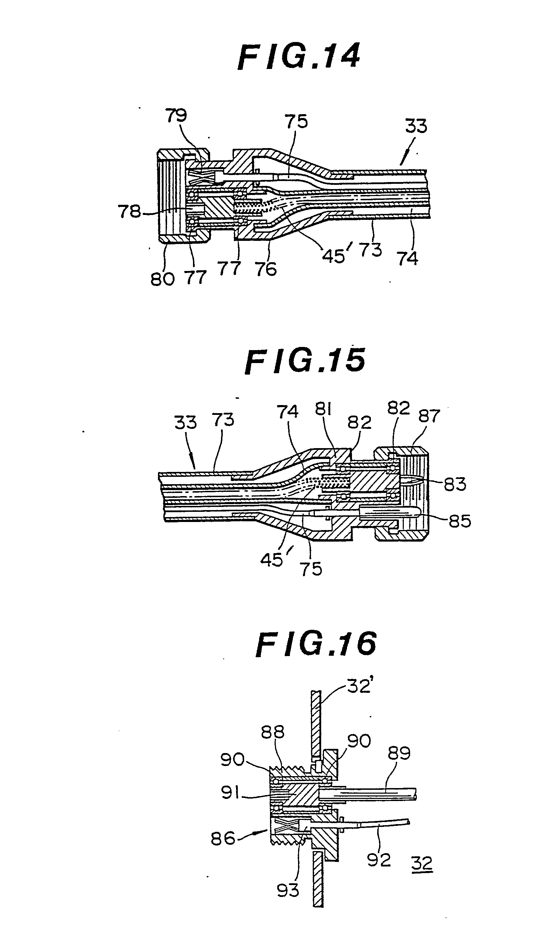

Further, as shown in Figures 14 and 15, the drive cable 33 is provided with a flexible drive shaft 45' inserted through a flexible tube 74, and also contains signal leads 75 for feeding the oscillator drive signals and receiving detector output signals, all within a flexible outer sleeve 73 of the drive cable 33. As shown in Figure 14, in the connecting part of the connector 67 at the grip end of the cable 33, a case 76 is fixed to the end of the outer sleeve 73, and the flexible tube 74 is secured to this case 76, which carries a drive shaft recess joint 78 borne by bearings 77, and female connecting pins 79 for the wiring 75. This case is provided with a screw-threaded rotatable connector flange 80 to be screwed to the connector 67 of the grip 29 after the drive shaft projection 72 of the connector 67 is engaged with the drive shaft recess joint 78 and the male connecting pins 68 of the connector 67 are engaged and connected with the female electric connecting pins 79 to complete the signal paths. As shown in Figure 15, at the driving means end of this drive cable 33, a case 81 is fixed to the outer sleeve 73, the flexible tube 74 is connected to this case 81, and a hexagonal drive shaft joint 83 carried in bearings 82 is provided, together with male connecting pins 85 to connect the wiring 75. This case 81 is provided with a rotatable screw-threaded connector 87 to engage with a connector 86 of the driving means 32 shown in Figure 16. The connector 86 of the driving means 32 shown in Figure 16 is fitted to a housing 32' and has a connector member 88 provided with a drive shaft recess 91, to connect the drive from a drive shaft 89 that is connected to a motor through a reduction mechanism or the like, the connector member 88 being carried by bearings 90, and female connecting pins 93 being provided to connect signal wiring 92. A male screw thread is formed on the outer periphery to engage with the connector 87 of the cable 33.

In Figure 13, a curvature operating lever 31 is indicated schematically on an operating link 94 leading to an operating unit 95 which moves the operating wire 48 that is connected to control the curving part 24.

With such a formation, then as is shown in Figure 11, the grip 29 can be held with one hand by the operator and the sheath 21 inserted into a body cavity, for example, an abdominal cavity through a trocar (not illustrated) with the inside of the thumb applied to the curvature operating lever 31 of the substantially spherical curve-control member 30 formed at the head of the grip 29. In such case, the probe tip 23 is guided to an internal organ while, for example, the diagonal front in the right direction of the sheath 21 is being observed via the perspective endoscope 27 from the rear of the curving part 24. After the location of the internal organ 49 is confirmed, the curvature operating lever 31 is operated by thumb-pressure to pull the operating wire 48 and cause the curving part 24 to curve in the direction of the field of view of the endoscope 27, to the right as seen from the operator when dealing with the abdominal cavity interior, and thus the probe tip 23 and window 22 are brought into the field of view and the window 22 can be viewed as it is brought into close contact with a predetermined part of the internal organ 49, as shown in Figure 12.

As the endoscope 27 is made to fit up to the inset window 25 formed in the sheath 21 to the rear of the curving part 24 and has a perspective view to the right of the tip, the probe tip 23 and curving part 28 can be inserted without intercepting the field of view to the internal organ, and when the probe tip 23 is moved into the field of view of the endoscope 27 by the action of the curving part 24, the operation of close contacting the internal organ 49 can be effected while both the internal organ 49 and probe tip 23 are being viewed, so that the internal organ can be accurately and easily examined. Also, the position of contact of the window 22 for transmitting and receiving ultrasonic waves with relation to the internal organ can be confirmed.

In the detailed embodiment the endoscope 27 is made as a separate unit that can be telescopically inserted into the insertion sheath 21, so that other available fine diameter endoscopes can be used, and an endoscope can be repaired or replaced in the event of failure. In alternative embodiments the endoscope may be integrally contained in the insertion sheath, to avoid the need to separately sterilise the endoscope tube or parts.

Our co-pending European Patent Application No. 82 301 453.5 (Specification No. 0 061 332) claims some of the subject matter disclosed herein.

## Claims

1. A combined endoscope and ultrasonic diagnostic device wherein the endoscope (27), provided with illuminating light from a light source (39) via a flexible light guide coupling (38), connected to extend laterally from the endoscope, is contained within an elongate insertion sheath (21) carrying an ultrasonic wave probe (23) for inserting directly into a body cavity and transmitting and receiving ultrasonic waves to facilitate diagnosis within the body cavity, the endoscope entering up to a bendable curving part (24), and the probe extending beyond the bendable curving part (24) and being provided with an ultrasonic oscillator (41) and a scanning mirror (42), the oscillator being connected via a signal lead (43) to drive means in a hand grip (29) adjacent the proximal end of the device, and the scanning mirror mounted on a rotary drive shaft (44) extending to the hand grip, on which is provided a control member (30) with a thumb-operable curvature control lever (31), characterised in that separate driving means (32) for the oscillator and scanning mirror are coupled to the control member (30) via a flexible drive cable coupling (33), whereby diagnosis can be facilitated by optical observation via the endoscope whilst using the ultrasonic probe, the flexible couplings (33, 38) enabling the hand grip itself to be relatively small and light, thereby improving the degree of positional control and reducing operator fatigue, and the flexible drive cable coupling (33) being connected to the control member (30) from the same lateral direction as the flexible light guide coupling (38) is connected to the endoscope (27) from the light source (39), to facilitate insertion and operation of the device.

2. A device as claimed in Claim 1, characterised in that said endoscope is integral with said insertion sheath.

3. A device as claimed in Claim 1, characterised in that said endoscope is removable from said insertion sheath.

## Revendications

1. Appareil de diagnostic constitué par la combinaison d'un endoscope et d'un dispositif à ultrasons, combinaison dans laquelle l'endoscope (27), pourvu d'une lumière éclairante provenant d'une source lumineuse (39), à travers un couplage (38) guide-lumière souple, connecté de façon à sortir latéralement de l'endoscope, est renfermé dans une gaine d'insertion (21) de forme allongée portant une probe (23) d'onde ultrasonique pour l'insertion directe dans une cavité du corps, et transmission et réception d'ondes ultrasoniques pour faciliter le diagnostic à l'intérieur de la cavité du corps, l'endoscope se terminant en une partie courbe pliable (24), et la probe s'étendant sous la partie courbe pliable (24) et étant munie d'un oscillateur ultrasonique (41) et d'un miroir d'exploration (42), l'oscillateur étant relié à travers un conduit de signal (43) à des organes de commande situés dans une poignée (29), adjacente à l'extrémité proche du dispositif, et le miroir d'exploration monté sur un arbre de commande rotatif (44) s'étendant jusqu'à la poignée, sur laquelle est prévu un organe de commande (30) avec un levier de commande (31) arrondi actionnable par le pouce, appareil caractérisé en ce que des organes de commande séparés (32) de l'oscillateur et du miroir d'exploration sont couplés à l'organe de commande (30) à travers un couplage (33) constitué par un câble de commande souple, le diagnostic pouvant être facilité par observation optique à travers l'endoscope pendant l'utilisation de la probe ultrasonique, les couplages souples (33, 38) permettant que la poignée elle-même soit relativement petite et légère, améliorant ainsi le degré de contrôle de position et réduisant la fatigue de l'opérateur, et le couplage (33) à câble de commande souple étant relié à l'organe de commande (30) suivant la même direction latérale que celle suivant laquelle le couplage guide-lumière souple (38) est relié à l'endoscope (27) à partir de la source lumineuse (29) afin de faciliter l'insertion et l'actionnement de l'appareil.

2. Appareil selon la revendication 1, caractérisé en ce que l'endoscope est solidaire de la gaine d'insertion.

3. Appareil selon la revendication 1, caractérisé en ce que l'endoscope est amovible de la gain d'insertion.

## Patentansprüche

1. Eine Verbindung eines Endoskops mit einem Ultraschalldiagnostikgerät, worin das Endoskop (27), versehen mit einer Beleuchtung von einer Lichtquelle (39) über eine biegsame Lichtleitverbindung (38), die so angebracht ist, dass sie sich seitwärts von dem Endoskop erstreckt, innerhalb eines langgestreckten Einsatzmantels (21) enthalten ist, der eine Ultraschall-Sonde (23) für direkte Einführung in eine Körperöffnung sowie zum Senden und Empfangen von Ultraschall-Wellen trägt, um eine Diagnose innerhalb der Körperöffnung zu erleichtern, wobei das Endoskop bis zu einem biegsamen Kurventeil (24) eindringt und die Sonde sich über den Kurventeil (24) erstreckt und mit einem Ultraschall-Oszillator (41) sowie einem Abtastspiegel (42) versehen ist, wobei der Oszillator über eine Signalleitung (43) mit Antriebsmitteln in einem Handgriff (29) verbunden ist, nahe dem proximalen Ende der Geräteverbindung, und der Abtastspiegel auf einer drehbaren Antriebswelle (44) angebracht ist, die sich von dem Handgriff erstreckt, auf dem ein Kontrollteil (30) mit einem durch den Daumen betätigbaren Biegungskontrollhebel (31) angebracht ist, dadurch gekennzeichnet, dass getrennte Antriebsmittel (32) für den Oszillator und den Abtastspiegel über ein biegsames Antriebsverbindungskabel (33) mit dem Kontrollteil (30) verbunden sind, wodurch die Diagnose erleichtert werden kann durch optische Beobachtung über das Endoskop, während die Ultraschall-Sonde in Gebrauch ist, die biegsamen Verbindungen (33, 38) eine möglichst kleine und leichte Gestaltung des Handgriffs erlauben, wodurch der Grad der Lagekontrolle verbessert und zu grosse Anstrengung des Benutzers vermieden wird, und das biegsame Antriebsverbindungskabel (33) mit dem Kontrollteil (30) verbunden ist in der gleichen seitlichen Richtung, in der die biegsame Lichtleitverbindung (38) und das Endoskop (27) von der Lichtquelle (39) angeschlossen ist, um das Einsetzen und die Betätigung der Geräteverbindung zu erleichtern.

2. Eine Geräteverbindung demäss Anspruch 1, dadurch gekennzeichnet, dass das genannte Endoskop mit dem genannten Einsatzmantel integral ist.

3. Eine Geräteverbindung gemäss Anspruch 1, dadurch gekennzeichnet, dass das genannte Endoskop von dem genannten Einsatzmantel entfernt werden kann.

# FIG.1

# FIG.2

**FIG.3**

0 061 331

# FIG.4

# FIG.5

# FIG.6

# FIG.7

# FIG.8

# FIG.9

FIG.10

# FIG.11

# FIG.12

FIG.13

# FIG.14

# FIG.15

# FIG.16